# EUROPEAN PATENT SPECIFICATION

(11) **EP 3 874 288 B1**
(45) Date of publication and mention of the grant of the patent: **06.11.2024**
(21) Application number: 19886585.9
(22) Date of filing: 20.11.2019
(51) Int. Cl.: G01R 33/483, G01R 33/48

(54) **A METHOD TO MEASURE TISSUE TEXTURE USING NMR SPECTROSCOPY WITH VOI LENGTH IN AN ANALYSIS DIRECTION DEFINED BY RECEIVER BANDWIDTH**
VERFAHREN ZUR MESSUNG DER GEWEBETEXTUR MITTELS NMR-SPEKTROSKOPIE MIT VOI-LÄNGE IN EINER DURCH EMPFÄNGERBANDBREITE DEFINIERTEN ANALYSERICHTUNG
PROCÉDÉ DE MESURE DE TEXTURE TISSULAIRE À L'AIDE D'UNE SPECTROSCOPIE PAR RÉSONANCE MAGNÉTIQUE NUCLÉAIRE (RMN) AVEC UNE LONGUEUR DE VOLUME D'INTÉRÊT (VOI) DANS UNE DIRECTION D'ANALYSE DÉFINIE PAR UNE BANDE PASSANTE DE RÉCEPTEUR

(30) Priority: 20.11.2018 US 201862769666 P
(43) Date of publication of application: 08.09.2021
(73) Proprietor: Bioprotonics, Inc., Santa Barbara, CA 93101 (US)
(72) Inventor: JAMES, Timothy, W., Santa Barbara, CA 93101 (US)
(74) Representative: Smith, Jeremy Robert
(86) International application number: PCT/US2019/062435
(87) International publication number: WO 2020/106857

(56) References cited:
- US-A1- 2015 241 537
- US-A1- 2017 030 986
- US-A1- 2017 030 986
- US-A1- 2017 108 567
- US-A1- 2018 238 986

## Description

### FIELD

The herein claimed method relates to the field of assessment using magnetic resonance (MR) of fine textures in biological systems, and in material and structural evaluation in industry and in engineering research. More specifically, the embodiments disclosed herein provide methods for selective excitation of a first slice within the specimen of interest and a second slice selective refocusing sequence to define a rod followed by application of a gradient along an analysis direction sweeping through a small range of k-values with the receiver bandwidth set narrowly to delineate the length of the volume of interest (VOI).

### BACKGROUND

US 2017/0030986 A1 describes selective sampling for assessing structural spatial frequencies with specific contrast mechanisms.

### SUMMARY

An invention is set out in claim 1. The invention provides a method for selective sampling to assess tissue texture in a specimen using magnetic resonance (MR). A first RF pulse is transmitted with a first gradient chosen for first slice selection in the specimen. A second RF pulse is transmitted with application of a second gradient chosen for slice selective refocusing in a region defined by an intersection of the first slice and a second slice defining a rod within the specimen. An encoding gradient pulse is applied to induce phase wrap to create a spatial encode for a specific k-value and orientation. A low non-zero magnitude gradient is then applied acting as a time dependent phase encode to produce a time varying trajectory through 3D k-space of k-value encodes while simultaneously recording multiple sequential samples of the NMR RF signal at a sequence of k-values across a neighborhood proximate the specific k-value defined by height and pulse width of the non-zero magnitude gradient and setting receiver bandwidth narrowly enough to delineate a length of a VOI within the rod during the data sampling. The samples are then post processed at the sequence of k-values, recorded within a time span while the non-zero magnitude gradient is applied, to characterize the textural features of tissue in the VOI.

### BRIEF DESCRIPTION OF THE DRAWINGS

FIG. 1 discloses an example timing diagram of a pulse sequence for the claimed method showing the timing of a single excitation;
FIG. 2 is a representation of the excited rod or core in the specimen and the resulting selected VOI during data sampling; and,
FIG. 3 is a flow chart showing implementation of a method for texture evaluation in the structure of a specimen.

### DETAILED DESRIPTION

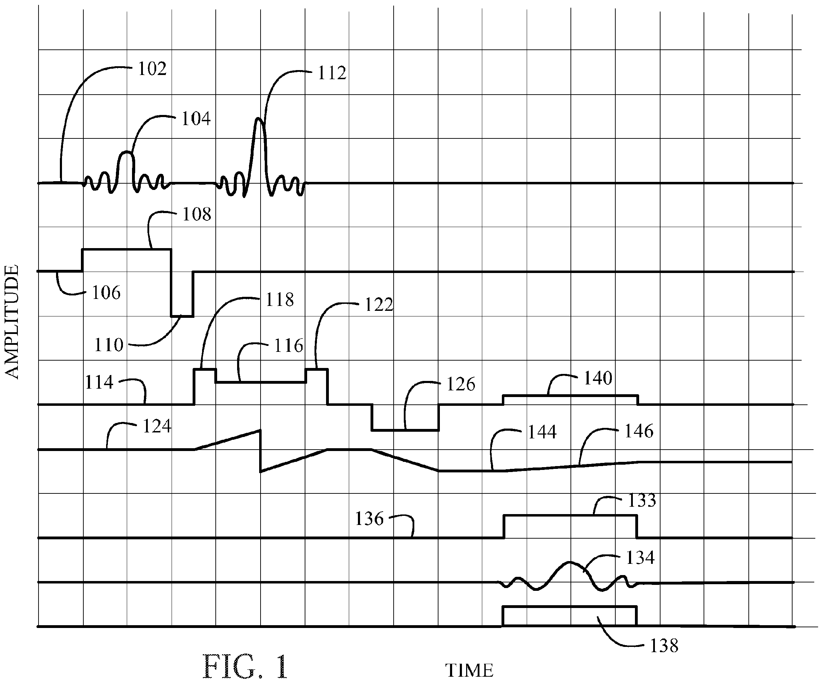

Referring to the drawings, FIG. 1 shows an example timing diagram for a pulse sequence for data acquisition using the method claimed herein. RF pulses included in trace 102 are employed to excite selected volumes of the tissue under investigation. A first RF pulse, 104, is transmitted coincidentally with a gradient pulse 108 on a first magnetic field gradient, represented in trace 106. This excites a single slice, or slab, of a specimen, the positioning of which is dependent on the orientation and magnitude of the first gradient, and the frequencies contained in the RF pulse. The negative gradient pulse, pulse 110, rephases the excitation within the defined thickness of the slice or slab.

A second RF pulse 112 is transmitted coincidentally with gradient pulse 116, on a second gradient, represented in trace 114, exciting a slice-selective refocus of spins, this second tissue slice intersecting with the first slice or slab described above. (As this second RF pulse 112 tips the net magnetic vector to antiparallel to B₀, it results in inversion of spins and subsequent refocusing, thus leading to a signal echo at a time after the 180 degree RF pulse equivalent to the time between the 90° and 180° RF pulses.) For the example shown, an initial higher value gradient pulse, 118, at the start of gradient pulse 116 is a crusher, or "spoiler" gradient, designed to induce a large phase wrap across the specimen volume. A similar gradient pulse, 122, at the trailing end of pulse 116, as it comes after the 180 degree RF inversion pulse 112, unwinds this phase wrap. In this way, any excitation that is not present prior to the 180 degree RF pulse, such as excitations from imperfections in the 180 pulse itself, will not have this pre-encode so will not be refocused by the second crusher, hence will not contribute to the signal. Note that the crushers 118 and 122 are shown in FIG. 1 as on only one axis. However, the crushers could be on any combination of axes.

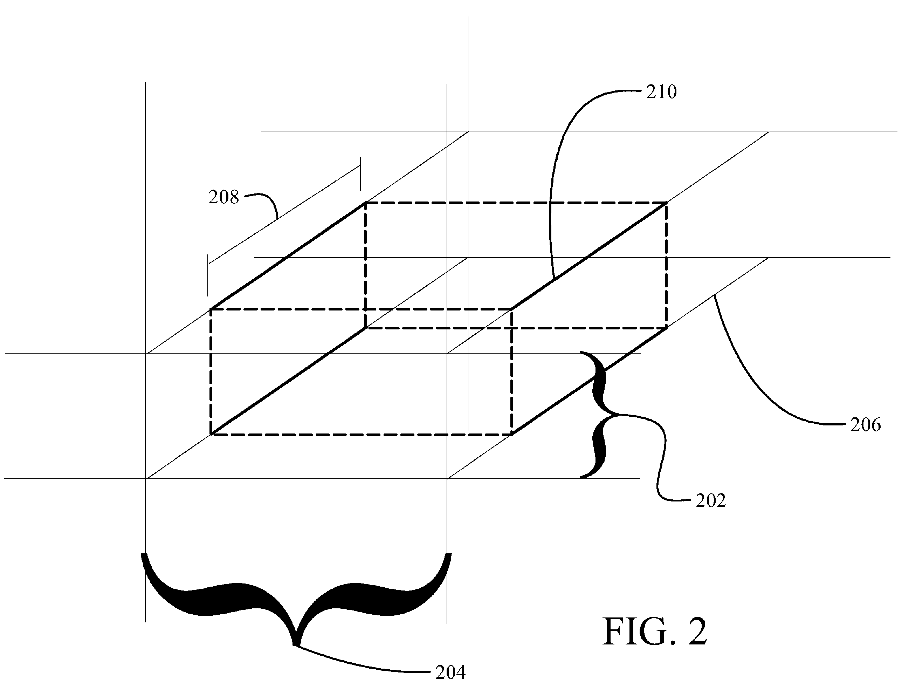

The second RF pulse, in combination with the applied second gradient, provides slice selective refocusing of the signal in a region defined by the intersection of the first slice 202 and the second slice 204 set by this second gradient thereby defining lateral dimensions of a rod or core 206 through the specimen as represented in FIG. 2. Those skilled in the art will recognize that there are a number of ways to generate an internally excited rod using time varying gradient pulses and RF excitations. Parameter selection for the various methods can be done with SNR optimization in mind. As described herein the rod or core 206 generated by a slice selective excitation and mutually- orthogonal slice selective refocusing pulses is represented in FIG. 2.

An encoding gradient pulse 126, on trace 114, sets an initial phase wrap, hence k-value encode, along the direction of gradient pulse 126. In general, the k-value encode can be oriented in any direction, by vector combination of the machine gradients but for ease of visualization is represented as on the second gradient. The negative encoding gradient pulse 126 winds up phase such that, in the signal echo signal acquisition starts at selected k-value, which may then be subsequently incremented or incremented or varied in orientation. While shown separated from second gradient pulse 116, the encoding gradient pulse 126 may be combined with pulse 122.

A low non-zero magnitude phase encode gradient 140 acting as a time dependent phase encode is applied and data samples 142 are taken from an initial k-value 144 for time varying k-values, seen in trace segment 146. The encode 140 for the initial k-value and the subsequent time varying k-values 146 do not need to be aligned with the axis of the excited rod. However, alignment of the phase encode gradient is aligned with the delineated direction of the VOI and, by definition aligned with an analysis direction, as described subsequently.

A receive gate 133 is opened to receive the RF signal, which is shown in FIG. 1 as pulse 134 on signal trace 136. The RF signal in trace 136 is a representation showing only the signal present in the receive gate window without showing the actual details of the RF signal outside the window. Sampling occurs as represented by trace 138 beginning with the initial k-value, 144, seen on trace 124. Note that, at the scale of the drawing, the sampling rate is high enough that the individual triggers of the analog to digital converter (A/D) have merged together in trace 138. The receiver bandwidth is set to delineate a length 208 of a VOI 210 within the rod or core 206 (seen in FIG. 2) during the data sampling. The initial phase wrap may be selected to provide an initial k-value with a magnitude corresponding to a low SNR region. The encoding gradient 126 may be employed to wind up to the lowest or highest k-value in a targeted texture and the non-zero magnitude gradient pulse is imposed in the necessary direction (increasing or decreasing k) to reach the other limit in k-space to define the texture. In an exemplary implementation a 50 ms read time (i.e., gradient on time) would be employed with a wavelength range 0.050 to 0.100 mm, Bzero = 1T, H1 nucleus (4.258MHz/T). A VOI analysis length 5mm could be obtained with a gradient of 23.5 mT/m and bandwidth of ~500 Hz. In practice the data would be acquired in a first VOI with a first significantly larger bandwidth with later data analysis and selection of multiple subset VOIs by post hoc selection of the bandwidth within the data, as described subsequently.

The echo can be refocused within the same excitation and again read with a phase encode gradient of the same magnitude as phase encode gradient 140 but in opposite direction to sweep back through the same range of k-values allowing implementation of phase cycling and achieving a higher SNR.

Using bandwidth to select the specimen length has the additional advantage that the band pass may be offset one way or another within the first bandwidth to access the sample in slightly different regions in the specimen along the same initially excited rod. As an example, the location of the width dimension 208 can also be set by the center frequency of the bandwidth chosen. This way multiple VOIs along the rod 206 defined by the first and second slice 202, 204 may be selected. This can be done by post processing the received broadband data set.

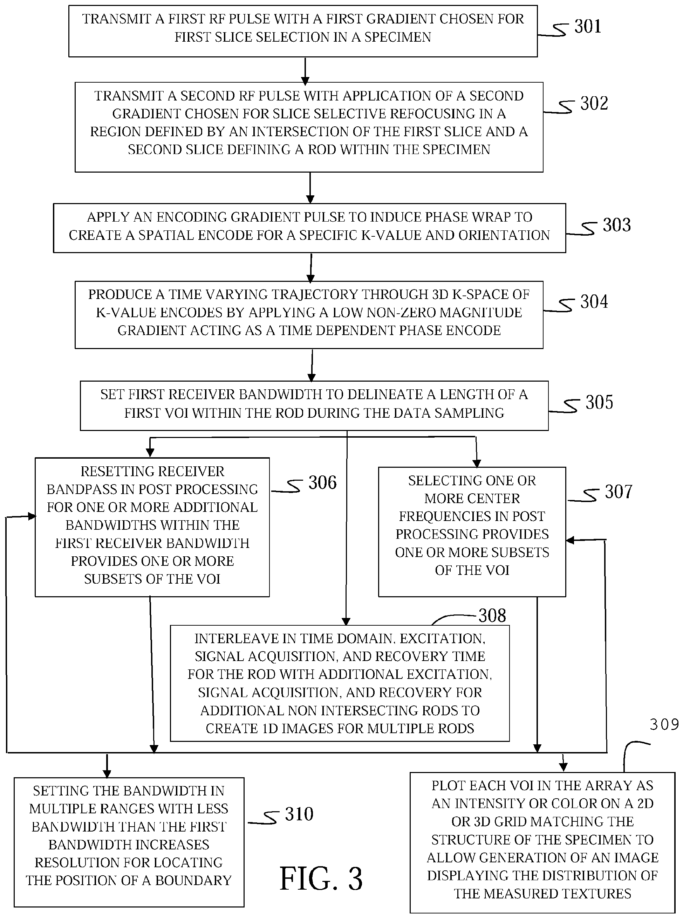

As shown in FIG. 3, an additional aspect of the disclosed method is iterating the disclosed method on an array of VOIs for a plurality of measured or derived values and plotting each VOI in the array as an intensity or color on a 2D or 3D grid matching the structure of the analyzed specimen to generate an image displaying the distribution of the measured textures. The method disclosed here is particularly well suited to efficiently gathering the multiplicity of VOI measurements. For each internally excited rod 206, setting a center frequency and sufficiently large bandwidth to cover the portion of the specimen intended for analysis for a first VOI can then be followed by repetitive selection of varying center frequency and bandwidth for a plurality of subset VOIs and post processing (filtering) along the axis of the gradient (rod 206) and, with each iteration, generating a 1D image of the measured (or derived) values. By interleaving in time domain, the excitation, signal acquisition, and recovery time for a rod with additional excitation, signal acquisition, and recovery for additional non intersecting rods, 1D images for multiple rods can also be efficiently acquired.

This additional aspect provides a sensitive method for locating boundaries of structures in the analyzed specimen for cases where the rod 206 intersects a boundary between differing texture types in the specimen. Setting the bandwidth in multiple narrow ranges within the first bandwidth during post processing has the effect of increasing the resolution for locating the position of the boundary.

Incorporating the excitation pulses and gradients as described with respect to FIG. 1, a first RF pulse is transmitted with a first gradient chosen for first slice selection in a specimen, step 301. A second RF pulse is transmitted with application of a second gradient chosen for slice selective refocusing in a region defined by an intersection of the first slice and a second slice defining a rod within the specimen, step 302. An encoding gradient pulse is applied to induce phase wrap to create a spatial encode for a specific k-value and orientation, step 303. By applying a low non-zero magnitude gradient acting as a time dependent phase encode a time varying trajectory through 3D k-space of k-value encodes is produced, step 304. A first receiver bandwidth is set to delineate a length of a first VOI within the rod during the data sampling, step 305, and multiple sequential samples of the NMR RF signal are simultaneously recorded at a sequence of k-values across a neighborhood proximate the specific k-value defined by height and pulse width of the non-zero magnitude gradient, step 305. The samples of the sequence of k values, recorded within a time span while the non-zero magnitude gradient was applied, are then post processed. Resetting receiver bandpass for one or more additional bandwidths within the first receiver bandwidth provides one or more subsets of the VOI, step 306. Alternatively, or in combination, one or more alternative center frequencies within the first receiver bandwidth are selected to provide one or more subsets of the VOI, step 307. By interleaving in time domain, excitation, signal acquisition, and recovery time for the rod with additional excitation, signal acquisition, and recovery for additional non intersecting rods, 1D images for multiple non-intersecting rods are created, step 308. By iterating for a selected set of pass bands within the first bandwidth to provide an array of subset VOIs for a plurality of measured or derived values, each subset VOI in the array may be plotted as an intensity or color on a 2D or 3D grid matching the structure of the specimen to allow generation of an image displaying the distribution of the measured textures, step 309. For identifying boundaries in measured textures in the specimen, setting the bandwidth in multiple ranges with less bandwidth than the first bandwidth increases resolution for locating the position of the boundary, step 310.

Having now described various embodiments of the invention in detail as required by the patent statutes, those skilled in the art will recognize modifications and substitutions to the specific embodiments disclosed as being within the scope of the present invention provided they fall under the scope of the following claims.

## Claims

1. A method for selective sampling to assess tissue texture in a specimen using magnetic resonance (MR)
comprising:
transmitting (301) a first RF pulse (104) with a first gradient chosen for first slice (202) selection in the specimen;
transmitting (302) a second RF pulse (112) with application of a second gradient chosen for slice selective refocusing in a region defined by an intersection of the first slice and a second slice (204) defining a rod (206) within the specimen;
applying (303) an encoding gradient pulse (126) to induce phase wrap to create a spatial encode for a specific k-value and orientation;
applying a low non-zero magnitude gradient (140) having a first magnitude acting as a time dependent phase encode to produce (304) a time varying trajectory through 3D k-space of k-value encodes, while
simultaneously recording multiple sequential samples of the NMR RF signal at a sequence of k-values across a neighborhood proximate the specific k-value defined by height and pulse width of the non-zero magnitude gradient in a single excitation;
setting (305) a first receiver bandwidth to delineate a length (208) of a volume of interest, VOI, (210) within the rod during the data sampling; and
post processing the samples at the sequence of k-values, recorded within a time span while the non-zero magnitude gradient is applied, to characterize the textural features of tissue(s) of the specimen in the VOI.

2. The method as defined in claim 1 further comprising:
refocusing;
applying a low non-zero magnitude gradient with a magnitude equal to but in an opposite direction to the first magnitude;
simultaneously recording multiple sequential samples of the NMR RF signal in a reverse of the sequence of k-values to sweep back through the same range of k-values allowing implementation of phase cycling.

3. The method as defined in claim 1 further comprising selecting (307) a center frequency of the first receiver bandwidth for positioning of the VOI (210) along the rod (206).

4. The method as defined in claim 3 wherein the step of post processing includes resetting (306) receiver bandpass for one or more additional bandwidths within the first receiver bandwidth providing one or more subsets of the VOI (210).

5. The method as defined in claim 3 wherein the step of post processing includes selecting (307) one or more alternative center frequencies within the first receiver bandwidth providing one or more subsets of the VOI (210).

6. The method as defined in claim 3 further comprising interleaving (308) in time domain, excitation, signal acquisition, and recovery time for the rod (206) with additional excitation, signal acquisition, and recovery for additional non intersecting rods, to create 1D images for multiple rods.

7. The method as defined in claim 1, wherein the step of post processing the samples further comprises:
iterating (306) a pass band within the first bandwidth providing an array of subset VOIs within the VOI for generating a plurality of measured or derived values; and
plotting (309) each subset VOI in the array as an intensity or color on a 2D or 3D grid matching the structure of the specimen to generate an image displaying the distribution of the measured textures corresponding to the measured or derived values.

8. The method as defined in claim 1,
wherein the step of setting the first receiver bandwidth to delineate the length (208) of the VOI (210) within the rod (206) during the data sampling further comprises setting the first receiver bandwidth to delineate the length of the VOI within the rod containing a boundary; and
wherein the step of post processing the samples at the sequence of k-values, recorded within the time span while the non-zero magnitude gradient was applied, further comprises setting (310) the bandwidth in multiple ranges with less bandwidth than the first receiver bandwidth thereby increasing resolution for locating the position of the boundary.

## Patentansprüche

1. Verfahren zur selektiven Probennahme zur Beurteilung der Gewebetextur in einer Probe mittels Magnetresonanz (MR), umfassend:
Senden (301) eines ersten HF-Impulses (104) mit einem ersten Gradienten, der für die Auswahl einer ersten Schicht (202) in der Probe gewählt wird;
Senden (302) eines zweiten HF-Impulses (112) unter Anwendung eines zweiten Gradienten, der für eine schichtselektive Refokussierung in einem Bereich gewählt wird, der durch den Schnitt der ersten Schicht und einer zweiten Schicht (204) definiert ist, wodurch ein Zylinder (206) innerhalb der Probe definiert wird;
Anwenden (303) eines Codiergradientenimpulses (126), um eine Phasenumwicklung zu induzieren und dadurch eine räumliche Codierung für einen spezifischen k-Wert und eine spezifische Orientierung zu erzeugen;
Anwenden eines niedrigen Gradienten (140) ungleich null mit einer ersten Gradientenstärke, der als zeitabhängige Phasencodierung wirkt, um eine zeitlich variierende Trajektorie durch den 3D-k-Raum von k-Wert-Codierungen zu erzeugen (304), während
gleichzeitig mehrere sequentielle Proben des NMR-HF-Signals bei einer Sequenz von k-Werten über eine Nachbarschaft nahe dem spezifischen k-Wert, der durch Höhe und Impulsbreite des Gradienten ungleich null definiert ist, in einer einzigen Anregung aufgezeichnet werden;
Einstellen (305) einer ersten Empfängerbandbreite, um eine Länge (208) eines Volumen von Interesse, VOI, (210) innerhalb des Zylinders während der Datenerfassung abzugrenzen; und
Nachverarbeiten der Proben bei der Sequenz von k-Werten, die innerhalb einer Zeitspanne aufgezeichnet wurden, während der Gradient ungleich null angewendet wird, um die Texturmerkmale des Gewebes/der Gewebe der Probe im VOI zu charakterisieren.

2. Verfahren nach Anspruch 1, ferner umfassend:
Refokussieren;
Anwenden eines niedrigen Gradienten ungleich null mit einer Gradientenstärke gleich, aber in entgegengesetzter Richtung zur ersten Gradientenstärke;
gleichzeitiges Aufzeichnen mehrerer sequentieller Proben des NMR-HF-Signals in einer umgekehrten Reihenfolge der k-Werte, um denselben Bereich von k-Werten rückwärts zu durchlaufen, wodurch die Implementierung von Phasenzyklen ermöglicht wird.

3. Verfahren nach Anspruch 1, ferner umfassend Auswählen (307) einer Mittenfrequenz der ersten Empfängerbandbreite zur Positionierung des VOI (210) entlang des Zylinders (206).

4. Verfahren nach Anspruch 3, wobei der Schritt des Nachverarbeitens das Zurücksetzen (306) des Empfängerbandpasses für eine oder mehrere zusätzliche Bandbreiten innerhalb der ersten Empfängerbandbreite beinhaltet, wodurch eine oder mehrere Teilmengen des VOI (210) bereitgestellt werden.

5. Verfahren nach Anspruch 3, wobei der Schritt des Nachverarbeitens das Auswählen (307) einer oder mehrerer alternativer Mittenfrequenzen innerhalb der ersten Empfängerbandbreite beinhaltet, wodurch eine oder mehrere Teilmengen des VOI (210) bereitgestellt werden.

6. Verfahren nach Anspruch 3, ferner umfassend das zeitliche Verschachteln (308) im Zeitbereich von Anregung, Signalerfassung und Erholungszeit für den Zylinder (206) mit zusätzlicher Anregung, Signalerfassung und Erholung für weitere, sich nicht überschneidende Zylinder, um 1D-Bilder für mehrere Zylinder zu erzeugen.

7. Verfahren nach Anspruch 1, wobei der Schritt des Nachverarbeitens der Proben ferner umfasst:
Iterieren (306) eines Durchlassbandes innerhalb der ersten Bandbreite, wodurch ein Feld von Teilmengen-VOIs innerhalb des VOI bereitgestellt wird, um eine Vielzahl von gemessenen oder abgeleiteten Werten zu erzeugen; und
Darstellen (309) jedes Teilmengen-VOI im Feld als Intensität oder Farbe auf einem 2D- oder 3D-Raster, das der Struktur der Probe entspricht, um ein Bild zu erzeugen, das die Verteilung der gemessenen Texturen entsprechend den gemessenen oder abgeleiteten Werten anzeigt.

8. Verfahren nach Anspruch 1,
wobei der Schritt des Einstellens der ersten Empfängerbandbreite zur Abgrenzung der Länge (208) des VOI (210) innerhalb des Zylinders (206) während der Datenerfassung ferner das Einstellen der ersten Empfängerbandbreite umfasst, um die Länge des VOI innerhalb des Zylinders abzugrenzen, der eine Grenze enthält; und
wobei der Schritt des Nachverarbeitens der Proben bei der Sequenz von k-Werten, die innerhalb der Zeitspanne aufgezeichnet wurden, während der Gradient ungleich null angewendet wurde, ferner das Einstellen (310) der Bandbreite in mehreren Bereichen mit geringerer Bandbreite als der ersten Empfängerbandbreite umfasst, wodurch die Auflösung zur Lokalisierung der Position der Grenze erhöht wird.

## Revendications

1. Procédé d'échantillonnage sélectif pour évaluer une texture de tissu dans une éprouvette à l'aide de résonance magnétique (MR), comprenant :
la transmission (301) d'une première impulsion RF (104) avec un premier gradient choisi pour la sélection d'une première coupe (202) dans l'éprouvette ;
la transmission (302) d'une seconde impulsion RF (112) avec application d'un second gradient choisi pour re-focalisation sélective en coupe dans une région définie par une intersection de la première coupe et d'une seconde coupe (204) définissant une baguette (206) à l'intérieur de l'éprouvette ;
l'application (303) d'une impulsion de gradient de codage (126) pour entraîner un enroulement de phase pour créer un code spatial pour une valeur k et orientation spécifique ;
l'application d'un faible gradient d'amplitude non zéro (140) ayant une première amplitude servant de code de phase à dépendance temporelle pour produire (304) une trajectoire variant en temps à travers un espace k 3D de codes de valeur k, durant
l'enregistrement simultané de multiples échantillons séquentiels du signal RF NMR à une séquence de valeurs k dans tout un voisinage à proximité de la valeur k spécifique définie par une hauteur et largeur d'impulsion du gradient d'amplitude non zéro dans une seule excitation ;
le réglage (305) d'une première largeur de bande de réception pour délinéer une longueur (208) d'un volume d'intérêt, VOI, (210) à l'intérieur de la baguette durant l'échantillonnage de données ; et
le post-traitement des échantillons à la séquence de valeurs k, enregistrées dans les limites d'un laps de temps alors que le gradient d'amplitude non zéro est appliqué, pour caractériser les particularités texturales de tissu(s) de l'éprouvette dans le VOI.

2. Procédé tel que défini dans la revendication 1, comprenant en outre :
la re-focalisation ;
l'application d'un faible gradient d'amplitude non zéro avec une amplitude égale à, mais dans une direction opposée à, la première amplitude ;
l'enregistrement simultané de multiples échantillons séquentiels du signal RF NMR dans un inverse de la séquence de valeurs k pour effectuer un re-balayage à travers la même plage de valeurs k, permettant la mise en œuvre de cyclage de phase.

3. Procédé tel que défini dans la revendication 1, comprenant en outre la sélection (307) d'une fréquence centrale de la première largeur de bande de réception pour le positionnement du VOI (210) le long de la baguette (206).

4. Procédé tel que défini dans la revendication 3, dans lequel l'étape du post-traitement inclut le re-réglage (306) d'une bande passante de réception pour une ou plusieurs largeurs de bande supplémentaires à l'intérieur de la première largeur de bande de réception, fournissant un ou plusieurs sous-ensembles du VOI (210).

5. Procédé tel que défini dans la revendication 3, dans lequel l'étape du post-traitement inclut la sélection (307) d'une ou de plusieurs autres fréquences centrales à l'intérieur de la première largeur de bande de réception, fournissant un ou plusieurs sous-ensembles du VOI (210).

6. Procédé tel que défini dans la revendication 3, comprenant en outre l'entrelacement (308), en domaine temporel, d'une excitation, d'une acquisition de signal, et d'un temps de récupération pour la baguette (206) avec une excitation, une acquisition de signal, et une récupération supplémentaires pour des baguettes supplémentaires sans intersection, pour créer des images 1D pour de multiples baguettes.

7. Procédé tel que défini dans la revendication 1, dans lequel l'étape du post-traitement des échantillons comprend en outre :
l'itération (306) d'une bande passante à l'intérieur de la première largeur de bande, fournissant un réseau de VOIs de sous-ensemble à l'intérieur du VOI pour générer une pluralité de valeurs mesurées ou dérivées ; et
le traçage (309) de chaque VOI de sous-ensemble dans le réseau sous forme d'intensité ou de couleur sur une grille 2D ou 3D assortie à la structure de l'éprouvette pour générer une image affichant la distribution des textures mesurées correspondant aux valeurs mesurées ou dérivées.

8. Procédé tel que défini dans la revendication 1,
dans lequel l'étape du réglage de la première largeur de bande de réception pour délinéer la longueur (208) du VOI (210) à l'intérieur de la baguette (206) durant l'échantillonnage de données comprend en outre le réglage de la première largeur de bande de réception pour délinéer la longueur du VOI à l'intérieur de la baguette contenant une limite ; et
dans lequel l'étape du post-traitement des échantillons à la séquence de valeurs k, enregistrées dans les limites du laps de temps alors que le gradient d'amplitude non zéro était appliqué, comprend en outre le réglage (310) de la largeur de bande dans de multiples plages avec moins de largeur de bande que la première largeur de bande de réception, ainsi augmentant la résolution pour localiser la position de la limite.
